# EUROPEAN PATENT SPECIFICATION

(11) **EP 4 005 463 B1**
(45) Date of publication and mention of the grant of the patent: **16.10.2024**
(21) Application number: 20844040.4
(22) Date of filing: 09.07.2020
(51) Int. Cl.: A61B 1/045, A61B 1/00, A61B 1/31, A61B 1/005

(54) **ENDOSCOPE SHAPE DISPLAY CONTROL DEVICE, OPERATION METHOD FOR ENDOSCOPE SHAPE DISPLAY CONTROL DEVICE, AND OPERATION PROGRAM FOR ENDOSCOPE SHAPE DISPLAY CONTROL DEVICE**
VORRICHTUNG ZUR ENDOSKOPFORMANZEIGESTEUERUNG, BETRIEBSVERFAHREN FÜR EINE VORRICHTUNG ZUR ENDOSKOPFORMANZEIGESTEUERUNG UND BETRIEBSPROGRAMM FÜR EINE VORRICHTUNG ZUR ENDOSKOPFORMANZEIGESTEUERUNG
DISPOSITIF DE COMMANDE D'AFFICHAGE DE FORME D'ENDOSCOPE, PROCÉDÉ DE FONCTIONNEMENT POUR DISPOSITIF DE COMMANDE D'AFFICHAGE DE FORME D'ENDOSCOPE, ET PROGRAMME DE FONCTIONNEMENT POUR DISPOSITIF DE COMMANDE D'AFFICHAGE DE FORME D'ENDOSCOPE

(30) Priority: 22.07.2019 JP 2019134865
(43) Date of publication of application: 01.06.2022
(73) Proprietor: FUJIFILM Corporation, Tokyo 106-8620 (JP)
(72) Inventor: YUMBE, Hirona, Ashigarakami-gun, Kanagawa 258-8538 (JP)
(74) Representative: Dehns Germany Partnerschaft mbB
(86) International application number: PCT/JP2020/026919
(87) International publication number: WO 2021/014991

(56) References cited:
- EP-A1- 2 196 127
- WO-A1-2009/044580
- JP-A- 2000 083 889
- JP-A- 2000 175 862
- JP-A- 2006 288 752
- JP-A- H08 542
- JP-B2- 4 159 396
- US-A- 5 913 820

## Description

### Technical Field

The present disclosure relates to an endoscope shape display control device, an operation method of an endoscope shape display control device, and an operation program of an endoscope shape display control device.

### Background Art

An endoscope that observes a lower digestive tract, such as the large intestine, is known. The lower digestive tract is intricately bent, unlike an upper digestive tract, such as an esophagus, which extends relatively linearly. Therefore, the endoscope, which is inserted into the lower digestive tract of a subject, such as a patient, an insertion part of the endoscope is also intricately bent. In order to grasp a shape of the insertion part of the endoscope inserted into the subject, an endoscope shape detection device that detects the shape of the insertion part of the endoscope in the subject (for example, see JP2004-551A) is known.

The endoscope shape detection device disclosed in JP2004-551A has a function of acquiring information for imaging the shape of the insertion part in the subject by using magnetism and imaging the shape of the insertion part based on the acquired information. For example, a plurality of magnetic field generation elements are disposed at intervals in the insertion part of the endoscope. The endoscope shape detection device detects a magnetic field generated by each magnetic field generation element in the insertion part by a magnetic field detection element disposed outside the subject. Since intensity of the magnetic field of each detected magnetic field generation element represents a position of each part of the insertion part with respect to the magnetic field detection element outside the subject, the endoscope shape detection device disclosed in JP2004-55 1A uses information on the intensity of the magnetic field of each magnetic field generation element as the information for imaging the shape of the insertion part to generate an image showing the shape of the insertion part. Further, the generated image is displayed on a monitor and presented to a user, such as an operator (for example, a doctor).

For example, EP 2 196 127 A1 discloses an endoscope shape analysis apparatus, which includes a coordinates obtaining portion for obtaining coordinate values of an insertion portion; a storage portion for storing the coordinate values; a straight line setting portion for setting a first straight line and a second straight line, based on the coordinate values; a coordinate transformation portion for transforming coordinates of a previous second straight line, based on a positional relationship between a previous first straight line and the first straight line, to calculate a third straight line; a determination portion for determining whether there is an error in a position display from a positional relationship between the first straight line, the second straight line, and the third straight line; and a correction portion for correcting the second straight line.

### SUMMARY OF INVENTION

### Technical Problem

An imaging procedure in the endoscope shape detection device of JP2004-55 1A is performed as follows, for example. First, the endoscope shape detection device disclosed in JP2004-551A specifies the position of each part of the insertion part to generate a three-dimension model of the insertion part based on the information for imaging the shape of the insertion part (for example, the information on the intensity of the magnetic field described above). Then, a rendering process is performed on the three-dimension model of the insertion part to generate a two-dimension image to be displayed on the monitor. The two-dimension image is an image showing the shape of the insertion part as viewed from one viewpoint in which the three-dimension model of the insertion part can be optionally set, and in the following, is referred to as a shape display image.

In a case in which the insertion part of the endoscope is bent in a loop shape in the subject, different portions of the insertion part intersect with each other in the shape display image depending on the set viewpoint. JP2004-551A discloses that a shade line process or a hidden surface process is performed in a case of the rendering process in order to express front and rear of the intersection portions.

However, depending on a display magnification of the shape display image, the insertion part of the endoscope may be displayed in a thin line shape in the shape display image. Therefore, there is a problem that it is difficult for the user to instantaneously determine the front and rear of the intersection portion even in a case in which the shade line process or the hidden surface process is performed on the intersection portion.

### Disclosure of the present invention

The present invention provides an endoscope shape display control device, an operation method of an endoscope shape display control device, and an operation program of the endoscope shape display control device with the features of the independent claims. The present invention is capable of displaying a shape display image in which an overlapping condition of an insertion part is easily visually recognized in the shape display image in which a shape of the insertion part of an endoscope in a state of being inserted into a subject is displayed.

An aspect of the present invention relates to an endoscope shape display control device comprising at least one processor, in which the processor acquires information for imaging a shape of an insertion part of an endoscope inserted in a subject, and displays, in a case of generating a shape display image in which the shape of the insertion part is displayed based on the acquired information, the insertion part with a pattern added on a surface thereof in the shape display image.

In the endoscope shape display control device according to the aspect described above, the pattern may be added to an entire region of the insertion part displayed in the shape display image.

In addition, in the endoscope shape display control device according to the aspect described above, the pattern may be a gradation in which a color is changed continuously or stepwise from one of a distal end side or a proximal end side of the insertion part to the other thereof. In this case, the gradation may be changed from green to blue.

According to a non-claimed example, the pattern may be a stripe pattern.

According to a non-claimed example, the pattern may be a wire frame pattern.

In addition, in the endoscope shape display control device according to the aspect described above, the processor may perform a shading process on the insertion part in addition to the pattern.

In addition, in the endoscope shape display control device according to the aspect described above, the information for imaging the shape of the insertion part may be information based on magnetic field measurement data obtained by using a plurality of magnetic field generation elements or a plurality of magnetic field detection elements provided in the insertion part.

Another aspect of the present invention relates to an operation method of an endoscope shape display control device, the method comprising an information acquisition step of acquiring information for imaging a shape of an insertion part of an endoscope inserted in a subject, and an image generation step of generating a shape display image in which the shape of the insertion part is displayed based on the acquired information and displaying the insertion part with a pattern added on a surface thereof in the shape display image.

Still another aspect of the present invention relates to an operation program of an endoscope shape display control device, the program causing a computer to function as an information acquisition unit that acquires information for imaging a shape of an insertion part of an endoscope inserted in a subject, and an image generation unit that generates a shape display image in which the shape of the insertion part is displayed based on the acquired information, the image generation unit displaying the insertion part with a pattern added on a surface thereof in the shape display image.

### Advantageous Effects of Invention

According to the technology of the present disclosure, it is possible to provide an endoscope shape display control device, an operation method of an endoscope shape display control device, and an operation program of the endoscope shape display control device capable of displaying a shape display image in which an overlapping condition of an insertion part is easily visually recognized in the shape display image in which a shape of the insertion part of an endoscope in a state of being inserted into a subject is displayed.

### BRIEF DESCRIPTION OF DRAWINGS

Fig. 1 is an explanatory diagram showing a state of an endoscopic examination using an endoscope system.
Fig. 2 is a schematic view showing an overall configuration of the endoscope system.
Fig. 3 is a block diagram showing an electric configuration of the endoscope system.
Fig. 4 is an explanatory diagram showing a state in which a plurality of detection coils detect a magnetic field generated by a plurality of generation coils.
Fig. 5 is an explanatory diagram showing an example of magnetic field measurement data.
Fig. 6 is an explanatory diagram for describing a determination process by a determination unit and a position detection process of each detection coil by a position detection unit.
Fig. 7 is an explanatory diagram showing an example of a shape detection process of an insertion part.
Fig. 8 is a functional block diagram of an image generation unit.
Fig. 9 is a flowchart for describing a flow of a generation process of a shape display image by a display control unit.
Fig. 10 is an explanatory diagram showing an example of a 3D model generation process of the insertion part.
Fig. 11 is a diagram showing an example of a hue circle.
Fig. 12 is a diagram showing an example of a shape display image.
Fig. 13 is a diagram showing an example of the shape display image.
Fig. 14 is a diagram showing an example of the shape display image.
Fig. 15 is a diagram showing an example of the shape display image.
Fig. 16 is a diagram showing an example of the shape display image.

### DESCRIPTION OF EMBODIMENTS

### [Overall Configuration of Endoscope System]

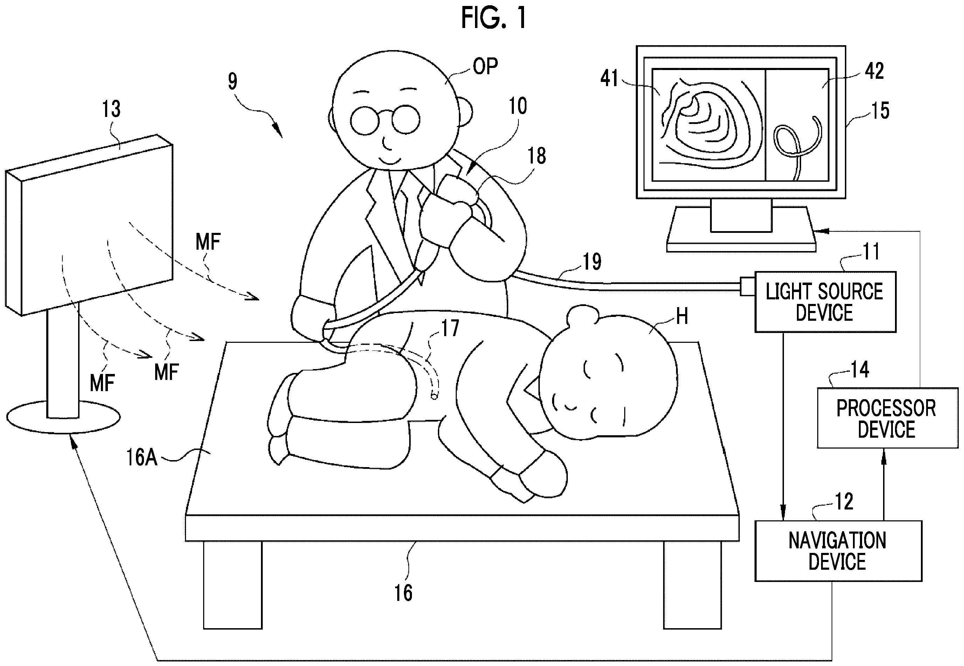

Fig. 1 is a schematic view showing an overall configuration of an endoscope system 9 comprising an endoscope shape display control device according to the technology of the present disclosure. As shown in Fig. 1, the endoscope system 9 comprises an endoscope 10, a light source device 11, a navigation device 12, a magnetic field generator 13, a processor device 14, and a monitor 15. The endoscope system 9 is used for an endoscopic examination in a body of a subject H, such as a patient. The subject H is an example of a subject. The endoscope 10 is, for example, an endoscope inserted into the digestive tract, such as the large intestine, and is a soft endoscope having flexibility. The endoscope 10 has an insertion part 17 to be inserted into the digestive tract, an operating part 18 connected sequentially to a proximal end side of the insertion part 17 and gripped by an operator to perform various operations, and a universal cord 19 connected sequentially to the operating part 18.

The endoscopic examination is performed, for example, in a state in which the subject H lies on an upper surface 16A of a bed 16. In a case of a large intestine examination, the insertion part 17 of the endoscope 10 is inserted into the digestive tract from an anus by an operator OP who is the doctor. The light source device 11 supplies illumination light that illuminates an inside of the large intestine, which is an observation site, to the endoscope 10. The processor device 14 generates an observation image 41 by processing an image captured by the endoscope 10. The observation image 41 is displayed on the monitor 15. The operator OP proceeds with the endoscopic examination while confirming the observation image 41. The observation image 41 displayed on the monitor 15 is basically a motion picture, but it is also possible to display a still picture as the observation image 41 as needed.

In addition, the endoscope system 9 has a navigation function of navigating technique, such as an insertion operation of the endoscope 10 performed by the operator OP. Here, the navigation means supporting the technique of the endoscope 10 of the operator OP by presenting an insertion state including the position and the shape of the insertion part 17 of the endoscope 10 in the body of the subject H to the operator OP. By the navigation function, the insertion state of the insertion part 17 is detected using a magnetic field MF and the detected insertion state is presented.

The navigation function is realized by the navigation device 12, the magnetic field generator 13, and a magnetic field measurement device in the endoscope 10 described below. The magnetic field generator 13 generates the magnetic field MF. The magnetic field generator 13 is attached to, for example, a stand and is disposed beside the bed 16 on which the subject H lies. In addition, the magnetic field generator 13 is disposed within a range in which the generated magnetic field MF reaches the body of the subject H.

The magnetic field measurement device in the endoscope 10 detects the magnetic field MF generated by the magnetic field generator 13 and measures the intensity of the detected magnetic field MF. The navigation device 12 detects the insertion state of the insertion part 17 by deriving a relative position between the magnetic field generator 13 and the insertion part 17 based on a magnetic field measurement result by the magnetic field measurement device. The processor device 14 generates a shape display image 42, which shows the insertion state detected by the navigation device 12.

The monitor 15 displays the observation image 41 and the shape display image 42. Note that the monitor 15 that displays the observation image 41 and the shape display image 42 may be provided separately.

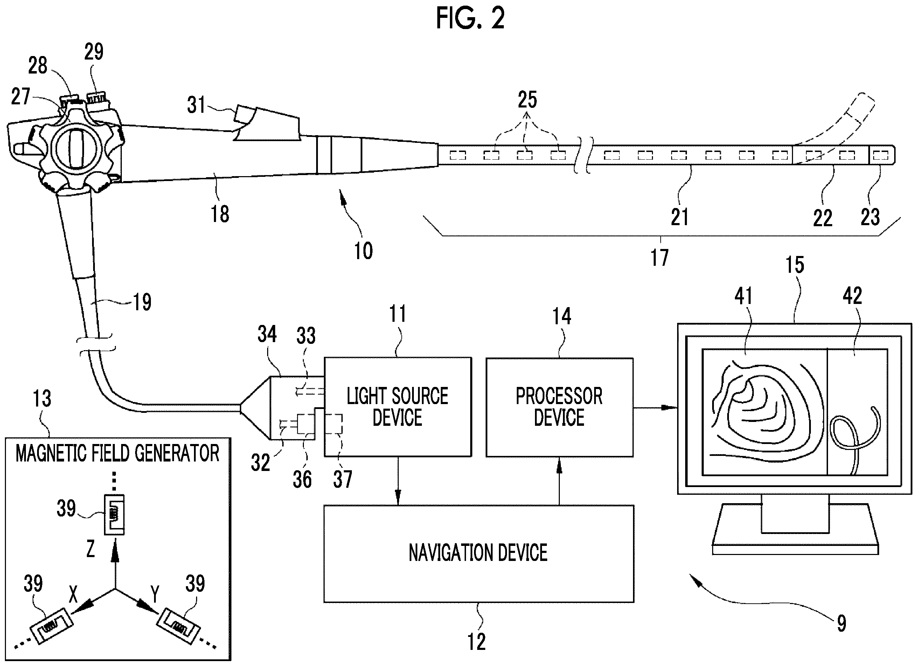

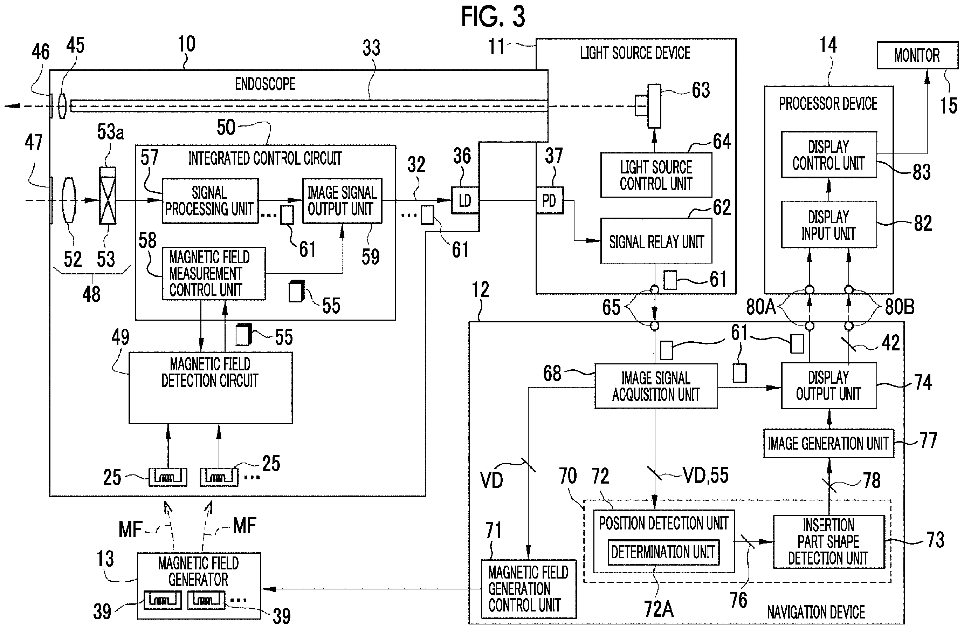

As shown in Fig. 2, the insertion part 17 is a tubular portion having a small diameter and a long length, and is configured by a soft part 21, a bendable part 22, and a distal end part 23, which are connected sequentially from the proximal end side to the distal end side. The soft part 21 has flexibility. The bendable part 22 is a part that can be bent by the operation of the operating part 18. An imaging apparatus 48 (see Fig. 3) and the like are disposed at the distal end part 23. In addition, although not shown in Fig. 2, an illumination window 46 (see Fig. 3) that illuminates the observation site with the illumination light, an observation window 47 (see Fig. 3) on which subject light of the illumination light reflected by the subject is incident, a treatment tool outlet (not shown) for a treatment tool to protrude, and a cleaning nozzle (not shown) for cleaning the observation window 47 by injecting gas and water into the observation window 47 are provided on a distal end surface of the distal end part 23.

Alight guide 33, a signal cable 32, an operation wire (not shown), and a pipe line for inserting the treatment tool (not shown) are provided in the insertion part 17. The light guide 33 extends from the universal cord 19 and guides the illumination light supplied from the light source device 11 to the illumination window 46 at the distal end part 23. The signal cable 32 is used for power supply to the imaging apparatus 48 in addition to communication of an image signal from the imaging apparatus 48 and a control signal for controlling the imaging apparatus 48. Like the light guide 33, the signal cable 32 also extends from the universal cord 19 and is arranged to the distal end part 23.

The operation wire is a wire for operating the bendable part 22, and is arranged from the operating part 18 to the bendable part 22. The pipe line for inserting the treatment tool is a pipe line for inserting the treatment tool (not shown), such as forcep, and is arranged from the operating part 18 to the distal end part 23. In addition, a fluid tube for air/water supply is provided in the insertion part 17. The fluid tube supplies the distal end part 23 with gas and water for cleaning the observation window 47.

In addition, in the insertion part 17, a plurality of detection coils 25 are provided from the soft part 21 to the distal end part 23 at preset intervals. Each detection coil 25 corresponds to a magnetic field detection element which detects the magnetic field MF. Each detection coil 25 is affected by the magnetic field MF generated from the magnetic field generator 13, so that an induced electromotive force is generated by an operation of electromagnetic induction, and an induced current is generated by the induced electromotive force. A value of the induced current generated from each detection coil 25 represents the intensity of the magnetic field MF detected by each detection coil 25, which is the magnetic field measurement result. That is, the magnetic field measurement result refers to a value depending on the magnitude of the induced current, which represents the intensity of the magnetic field MF.

The operating part 18 is provided with various operation members operated by the operator. Specifically, the operating part 18 is provided with two types of bending operation knobs 27, an air/water supply button 28, and a suction button 29. Each of the two types of bending operation knobs 27 is connected to the operation wire, and is used for a right-left bending operation and an up-down bending operation of the bendable part 22. In addition, the operating part 18 is provided with a treatment tool inlet port 31 which is an inlet of the pipe line for inserting the treatment tool.

The universal cord 19 is a connection cord that connects the endoscope 10 to the light source device 11. The universal cord 19 encompasses the signal cable 32, the light guide 33, and the fluid tube (not shown). In addition, a connector 34 connected to the light source device 11 is provided at an end part of the universal cord 19.

By connecting the connector 34 to the light source device 11, the light source device 11 supplies the power, the control signal, the illumination light, the gas, and the water necessary for operating the endoscope 10 to the endoscope 10. In addition, the image signal of the observation site acquired by the imaging apparatus 48 (see Fig. 2) of the distal end part 23 and the magnetic field measurement result based on a detection signal of each detection coil 25 are transmitted from the endoscope 10 to the light source device 11.

The connector 34 is not electrically connected to the light source device 11 by wire using a metal signal line or the like, and instead, the connector 34 and the light source device 11 are connected to each other so as to be capable of optical communication (for example, non-contact type communication). The connector 34 transmits and receives the control signal exchanged between the endoscope 10 and the light source device 11 and transmits the image signal and the magnetic field measurement result from the endoscope 10 to the light source device 11 by optical communication. The connector 34 is provided with a laser diode (hereinafter, referred to as LD) 36 connected to the signal cable 32.

The LD 36 is used for transmitting a large amount of data from the endoscope 10 to the light source device 11, specifically, transmitting the image signal and the magnetic field measurement result. The LD 36 transmits the image signal and the magnetic field measurement result, which has been originally in a form of the electric signal, in a form of an optical signal to a photodiode (hereinafter, referred to as PD) 37 provided in the light source device 11.

Note that, although not shown, apart from the LD 36 and the PD 37, both the connector 34 and the light source device 11 are provided with a light transmission and reception unit that converts a small amount of the control signal exchanged between the endoscope 10 and the light source device 11 into the optical signal and transmits and receives the converted optical signal. Moreover, the connector 34 is provided with a power reception unit (not shown) that receives the power by wireless power supply from a power feed unit (not shown) of the light source device 11.

The light guide 33 in the connector 34 is inserted into the light source device 11. In addition, the fluid tube (not shown) in the connector 34 is connected to an air/water supply device (not shown) via the light source device 11. As a result, the light source device 11 and the air/water supply device supply the illumination light, the gas, and the water to the endoscope 10, respectively.

The light source device 11 supplies the illumination light to the light guide 33 of the endoscope 10 via the connector 34, and supplies the gas and the water supplied from the air/water supply device (not shown) to the fluid tube (not shown) of the endoscope 10. In addition, the light source device 11 receives the optical signal transmitted from the LD 36 by the PD 37, converts the received optical signal into the original image signal, which is the electric signal, and the magnetic field measurement result, and then outputs the converted image signal and the magnetic field measurement result to the navigation device 12.

The navigation device 12 outputs the image signal for generating the observation image 41 input from the light source device 11 to the processor device 14. In addition, the navigation device 12 controls the drive of the magnetic field generator 13 described below, detects the shape and the like of the insertion part 17 in the body of the subject H, and outputs a detection result to the processor device 14 as the information for generating the shape display image 42.

As described above, the endoscope 10 in the present embodiment is one-connector type having one connector 34 connected to the light source device 11. The endoscope 10 is connected to each of the processor device 14 and the navigation device 12 so as to be capable of communication via the light source device 11 to which the connector 34 is connected.

The magnetic field generator 13 has a plurality of generation coils 39 corresponding to a plurality of magnetic field generation elements. Each generation coil 39 includes an X-axis coil, a Y-axis coil, and a Z-axis coil that generate, by applying a drive current, an alternating current magnetic field (in other words, an alternating current magnetic field) in directions corresponding to XYZ coordinate axes of an orthogonal coordinate system XYZ, respectively. Each generation coil 39 generates the magnetic field MF having the same frequency. The generation coils 39 generate the magnetic fields MF at different timings from each other under a control of the navigation device 12, which will be described in detail below.

### <Endoscope>

Fig. 3 is a block diagram showing an electric configuration of the endoscope system 9. As shown in Fig. 3, the endoscope 10 includes the light guide 33, an irradiation lens 45, the illumination window 46, the observation window 47, the imaging apparatus 48, a magnetic field detection circuit 49, an integrated control circuit 50, the signal cable 32, the LD 36, and the fluid tube and the cleaning nozzle (which are not shown).

The light guide 33 is, for example, a large-diameter optical fiber or a bundle fiber.

An incident end of the light guide 33 is inserted into the light source device 11 via the connector 34. The light guide 33 is inserted into the connector 34, the universal cord 19, and the operating part 18, and an emission end faces the irradiation lens 45 provided in the distal end part 23 of the insertion part 17. As a result, the illumination light supplied from the light source device 11 to the incident end of the light guide 33 is emitted to the observation site from the irradiation lens 45 through the illumination window 46 provided on the distal end surface of the distal end part 23. Further, the illumination light reflected by the observation site is incident on an imaging surface of the imaging apparatus 48 as image light of the observation site through the observation window 47 provided on the distal end surface of the distal end part 23.

Note that one end side of the fluid tube (not shown) described above is connected to the air/water supply device (not shown) through the connector 34 and the light source device 11, and the other end side of the fluid tube (not shown) is connected to an air/water supply nozzle (not shown) provided on the distal end surface of the distal end part 23 through the insertion part 17 and the like. As a result, the gas or the water supplied from the air/water supply device (not shown) is injected into the observation window 47 from the air/water supply nozzle (not shown) to clean the observation window 47.

The imaging apparatus 48 includes a condenser lens 52 and an imaging element 53. The condenser lens 52 collects the image light of the observation site incident from the observation window 47, and forms the collected image light of the observation site on the imaging surface of the imaging element 53. The imaging element 53 is a complementary metal oxide semiconductor (CMOS) type or charge coupled device (CCD) type imaging element. The imaging element 53 is, for example, a color imaging element in which any of red (R), green (G), or blue (B) microfilter is assigned to each pixel. The imaging element 53 images the observation site, which is an observation target. More specifically, the imaging element 53 images the image light of the observation site imaged on the imaging surface (that is, converts the image light into the electric signal), and outputs the image signal of the observation site to the integrated control circuit 50.

In addition, the imaging element 53 is provided with an oscillation unit 53a that outputs a reference signal (for example, a clock signal), such as a crystal oscillator, and the imaging element 53 outputs the image signal constituting the motion picture with the reference signal oscillated from the oscillation unit 53a as a reference. An interval of the reference signal defines a frame rate. The frame rate is, for example, 30 frames per second (fps).

The magnetic field detection circuit 49 is electrically connected to each detection coil 25 in the insertion part 17. The magnetic field detection circuit 49 outputs magnetic field measurement data 55 including the magnetic field measurement result of each detection coil 25 depending on the magnetic field MF generated from the generation coil 39 of the magnetic field generator 13 to the integrated control circuit 50.

The integrated control circuit 50 configured to include arithmetic circuits including various central processing units (CPU) and various memories, and controls the operations of the units of the endoscope 10 in an integrated manner. The integrated control circuit 50 functions as a signal processing unit 57, a magnetic field measurement control unit 58, and an image signal output unit 59 by executing a control program stored in a memory (not shown). The magnetic field detection circuit 49 and the magnetic field measurement control unit 58 are combined to configure a magnetic field measurement unit. The magnetic field measurement unit measures a plurality of the magnetic fields MF originating from the generation coils 39 corresponding to the plurality of magnetic field generation elements based on the detection signals output by the detection coils 25, and outputs the magnetic field measurement result for each magnetic field MF. The magnetic field measurement unit and the detection coil 25 are combined to configure the magnetic field measurement device.

The signal processing unit 57 performs various signal processes on the image signals sequentially output from the imaging element 53. The signal process includes, for example, an analog signal process, such as a sampling two correlation pile process and a signal amplification process, and an analog/digital (A/D) conversion process of converting an analog signal into a digital signal after the analog signal process. The image signal after the signal process is performed is called a frame image signal 61. The signal processing unit 57 outputs the frame image signal 61 to the image signal output unit 59 depending on the frame rate. The frame image signal 61 is used as motion picture data of the observation site. As described above, a plurality of the frame image signals 61 are the image signals that are acquired by the imaging element 53 executing motion picture imaging and are output at preset time intervals.

The magnetic field measurement control unit 58 acquires the magnetic field measurement data 55 including a plurality of the magnetic field measurement results of the detection coils 25 via the magnetic field detection circuit 49, and outputs the acquired magnetic field measurement data 55 to the image signal output unit 59.

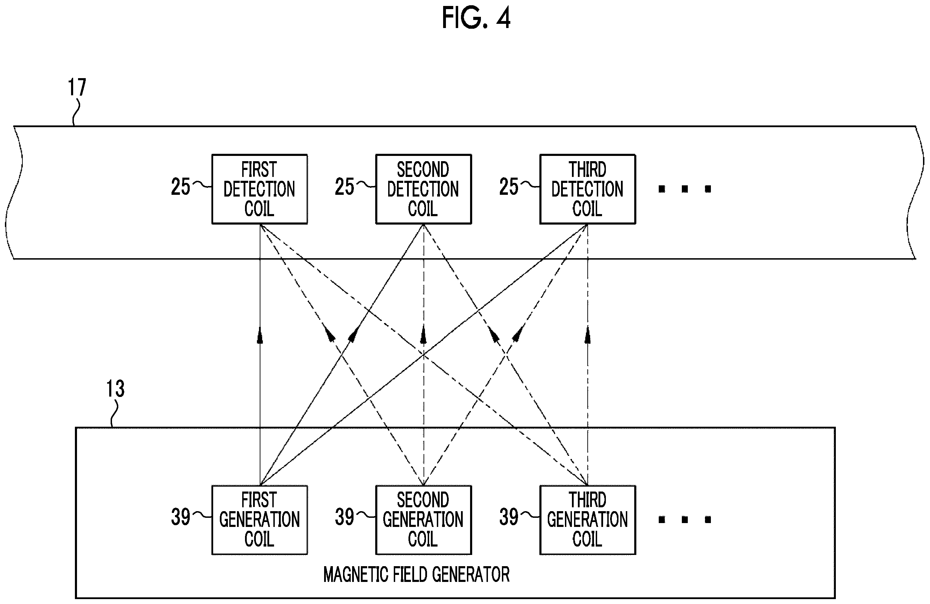

As shown in Fig. 4, even in a case in which the intensity of the magnetic field generated by each generation coil 39 is the same, for example, the magnetic field measurement result of each detection coil 25 is changed depending on a distance and a direction between each generation coil 39 that generates the magnetic field MF and each detection coil 25. For example, the first generation coil 39 shown in Fig. 4 has different distance and direction from each of the first to third detection coils 25, as shown by a solid line. Therefore, the magnetic field measurement results of the first to third detection coils 25 for the magnetic field MF generated by one first generation coil 39 are different. A relationship between each of the second generation coil 39 and the third generation coil 39 and each of the first to third detection coils 25 is the same.

In addition, on the contrary, even in a case in which the intensity of the magnetic field MF generated by each of the first to third generation coils 39 is the same, the magnetic field measurement result of one first detection coil 25 for the magnetic field MF of each generation coil 39 is different. Here, for example, a case is considered in which the first to third generation coils 39 is the X-axis coil, the Y-axis coil, and the Z-axis coil, respectively. In this case, a three-dimension coordinate position of the first detection coil 25 corresponding to the XYZ coordinate axes can be detected based on the magnetic field measurement result of one first detection coil 25 for the magnetic field MF of each of the X-axis coil, the Y-axis coil, and the Z-axis coil. The same applies to the second detection coil 25 and the third detection coil 25. In a case in which the three-dimension coordinate positions of the detection coils 25 provided in the insertion part 17 at preset intervals can be detected, the shape of the insertion part 17 can be detected.

Note that, actually, based on the magnetic field measurement result, an angle of each detection coil 25 is detected in addition to the three-dimension coordinate position of each detection coil 25. The shape of the insertion part 17 is detected based on the information of the three-dimension coordinate position and the angle. In the following, in order to avoid complication, the description regarding the angle will be omitted and only the three-dimension coordinate position will be described.

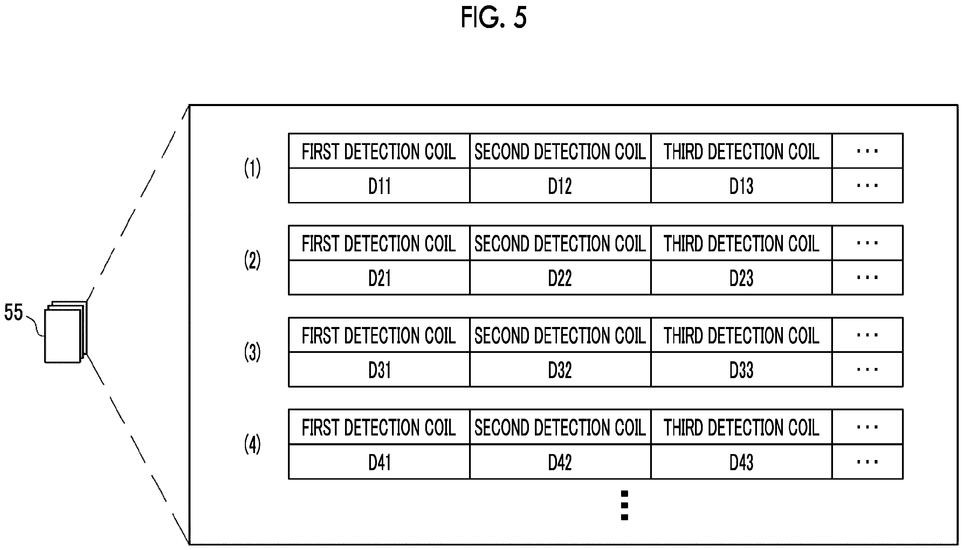

Fig. 5 is an explanatory diagram for describing an example of the magnetic field measurement data 55 acquired by the magnetic field measurement control unit 58. The magnetic field measurement control unit 58 detects the plurality of magnetic fields MF generated by the plurality of generation coils 39 by the plurality of detection coils 25 and acquires the magnetic field measurement data 55 including the plurality of magnetic field measurement results output from the detection coils 25.

In Fig. 5, (1) to (4) are data strings showing the magnetic field measurement results of the plurality of detection coils 25 with respect to the magnetic fields MF generated by the generation coils 39, respectively. For example, "D11" is a magnetic field measurement result in which the magnetic field MF generated by the first generation coil 39 is detected by the first detection coil 25. "D12" is a magnetic field measurement result in which the magnetic field MF generated by the first generation coil 39 is detected by the "second detection coil". Similarly, "D42" is a magnetic field measurement result in which the magnetic field MF generated by the fourth generation coil 39 is detected by the second detection coil 25. "D43" is a magnetic field measurement result in which the magnetic field MF generated by the fourth generation coil 39 is detected by the third detection coil 25.

The magnetic field measurement control unit 58 sequentially acquires the magnetic field measurement results of the detection coils 25 while synchronizing with a magnetic field generation timing of each generation coil 39 in the magnetic field generator 13. The magnetic field measurement control unit 58 acquires, for example, the magnetic field measurement results of all of the detection coils 25 for the magnetic fields MF of all of the generation coils 39 in one magnetic field measurement period defined by a synchronization signal described below. As a result, the magnetic field measurement control unit 58 acquires the magnetic field measurement data 55 including the plurality of magnetic field measurement results relating to all of combinations of the generation coils 39 and the detection coils 25 in one magnetic field measurement period.

For example, in a case in which 9 generation coils 39 are provided in the magnetic field generator 13 and 17 detection coils 25 are provided in the insertion part 17, 17 magnetic field measurement results are obtained for each generation coil 39. Therefore, the magnetic field measurement control unit 58 acquires the magnetic field measurement data 55 including a total of 9 × 17 = 153 magnetic field measurement results in one magnetic field measurement period. The magnetic field measurement data 55 including the plurality of magnetic field measurement results relating to all of such combinations is referred to as total magnetic field measurement data. In the present embodiment, unless otherwise specified, the magnetic field measurement data 55 shall include the total magnetic field measurement data.

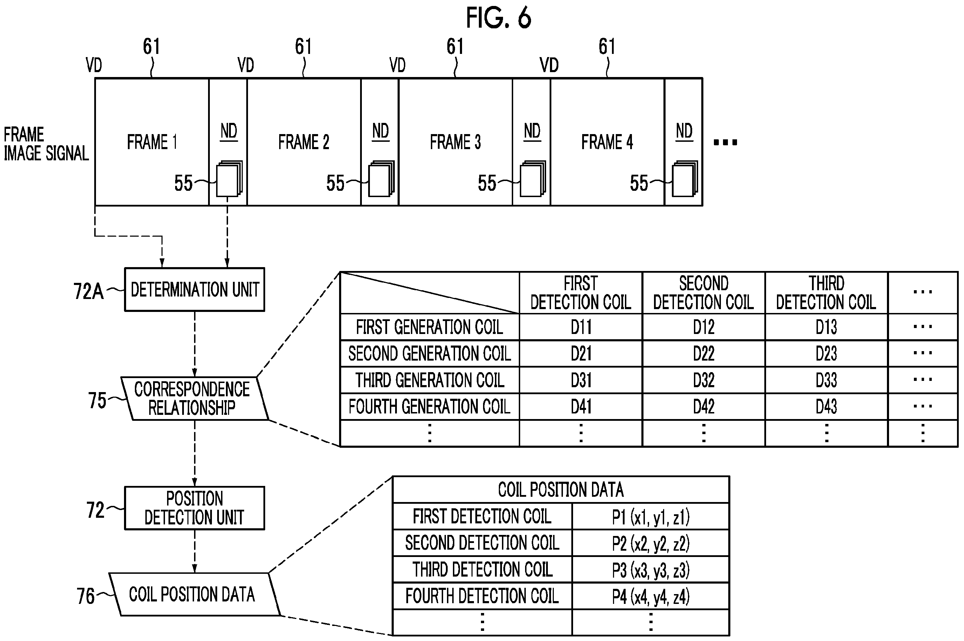

As shown in Fig. 6, the image signal output unit 59 adds a frame start signal VD to each of the plurality of frame image signals 61 sequentially input from the signal processing unit 57, and outputs the frame image signal 61. In Fig. 6, "frame 1", "frame 2", "frame 3" ... are frame numbers indicating output order of the plurality of frame image signals 61, which are shown for convenience. The frame start signal VD is a vertical synchronization signal, for example.

Moreover, the image signal output unit 59 adds the magnetic field measurement data 55 to the frame image signal 61 and outputs the frame image signal 61. That is, the frame start signal VD and the magnetic field measurement data 55 are included in all of the frame image signals 61 output by the image signal output unit 59. As shown in Fig. 6, the magnetic field measurement data 55 is added to a signal invalid region ND between the frame image signals 61, which corresponds to a blanking time of the imaging element 53. The blanking time is a vertical blanking period, for example. As described above, the frame start signal VD is also the signal included in the vertical blanking period of the plurality of frame image signals 61.

In Fig. 3, the image signal output unit 59 outputs the frame image signal 61 to the LD 36 described above via the signal cable 32. The LD 36 transmits the optical signal obtained by converting the frame image signal 61 into the optical signal to the PD 37 of the light source device 11.

As described above, the image signal output unit 59 outputs the frame image signal 61 acquired from the imaging element 53 via the signal processing unit 57 to the outside of the endoscope 10. In addition, by using the frame start signal VD included in the frame image signal 61 as the synchronization signal, the image signal output unit 59 functions as a synchronization signal generation unit.

### <Light Source Device>

The light source device 11 includes an illumination light source 63, the PD 37, a light source control unit 64, a signal relay unit 62, and a communication interface 65. The illumination light source 63 is configured to include a semiconductor light source, such as the laser diode (LD) or a light emitting diode (LED), and is a white light source which emits white light having a wavelength range from a red region to a blue region as the illumination light. Note that, as the illumination light source 63, in addition to the white light source, a special light source that emits special light, such as purple light and infrared light, may be used. The illumination light emitted from the illumination light source 63 is incident on the incident end of the light guide 33 described above.

The light source control unit 64 is configured to include various arithmetic circuits including the CPU and various memories, and controls the operation of each unit of the light source device 11, such as the illumination light source 63.

The PD 37 receives the optical signal transmitted from the LD 36. The PD 37 converts the frame image signal 61 received in the form of the optical signal into the form of the original electric signal, and inputs the converted frame image signal 61 to the signal relay unit 62. The signal relay unit 62 is configured by, for example, a field programmable gate array (FPGA). The FPGA is a processor of which a circuit configuration can be changed after manufacturing, and the circuit configuration includes various arithmetic circuits and various memory circuits.

### <Navigation Device>

The navigation device 12 includes an image signal acquisition unit 68, an insertion state detection unit 70, a magnetic field generation control unit 71, a display output unit 74, and an image generation unit 77. Each unit of the navigation device 12 is configured by various arithmetic circuits (not shown) including one or a plurality of CPUs, and is operated by executing a control program stored in a memory (not shown).

The image signal acquisition unit 68 acquires the frame image signal 61 from the signal relay unit 62 via the communication interface 65. Further, the image signal acquisition unit 68 outputs the acquired frame image signal 61 to the display output unit 74.

In addition, the image signal acquisition unit 68 extracts the frame start signal VD and the magnetic field measurement data 55 included in the frame image signal 61, and outputs the extracted frame start signal VD and the magnetic field measurement data 55 to the insertion state detection unit 70. In addition, the image signal acquisition unit 68 extracts the frame start signal VD from the frame image signal 61, and outputs the extracted frame start signal VD to the magnetic field generation control unit 71.

The insertion state detection unit 70 detects the insertion state of the insertion part 17 of the endoscope 10 inserted into the body of the subject H based on the frame start signal VD and the magnetic field measurement data 55 acquired from the image signal acquisition unit 68. The insertion state detection unit 70 includes a position detection unit 72 and an insertion part shape detection unit 73.

The position detection unit 72 detects a position of each detection coil 25 based on the frame start signal VD and the magnetic field measurement data 55. A determination unit 72A is provided in the position detection unit 72.

As shown in Fig. 6, the determination unit 72A determines the plurality of magnetic field measurement results included in the magnetic field measurement data 55 with reference to a correspondence relationship 75. The correspondence relationship 75 is information indicating storage order of the plurality of magnetic field measurement results corresponding to a plurality of combinations of the generation coils 39 and the detection coils 25 included in the magnetic field measurement data 55. The determination unit 72A determines which combination of each generation coil 39 and each detection coil 25 corresponds to each magnetic field measurement result included in the magnetic field measurement data 55 based on the correspondence relationship 75.

Specifically, as will be described below, in the magnetic field measurement, generation order in which the generation coils 39 generate the magnetic field MF with the frame start signal VD as a reference and acquisition order of the magnetic field measurement results of the detection coils 25 for the magnetic field MF of one generation coil 39 are decided. The plurality of magnetic field measurement results corresponding to the combinations of the generation coils 39 and the detection coils 25 are stored in the magnetic field measurement data 55 depending on the generation order and the acquisition order. Therefore, the determination unit 72A can determine which combination of the plurality of magnetic field measurement results included in the magnetic field measurement data 55 (for example, "D11", "D12", "D13", ...) corresponds to each magnetic field measurement result by referring to the correspondence relationship 75 that defines the storage order with the frame start signal VD as a reference.

The position detection unit 72 detects, as coil position data, the position of each detection coil 25, specifically, the three-dimension coordinate position based on the plurality of magnetic field measurement results determined by the determination unit 72A. The coil position data is a relative position with the magnetic field generator 13 as a reference. In Fig. 6, for example, P1 indicates the three-dimension coordinate position (here, x1, y1, z1) of the first detection coil 25. The same applies to P2, P3, P4, and the like.

In Fig. 3, the position detection unit 72 outputs coil position data 76 to the insertion part shape detection unit 73. The insertion part shape detection unit 73 detects the shape of the insertion part 17 in the body of the subject H based on the coil position data 76 input from the position detection unit 72.

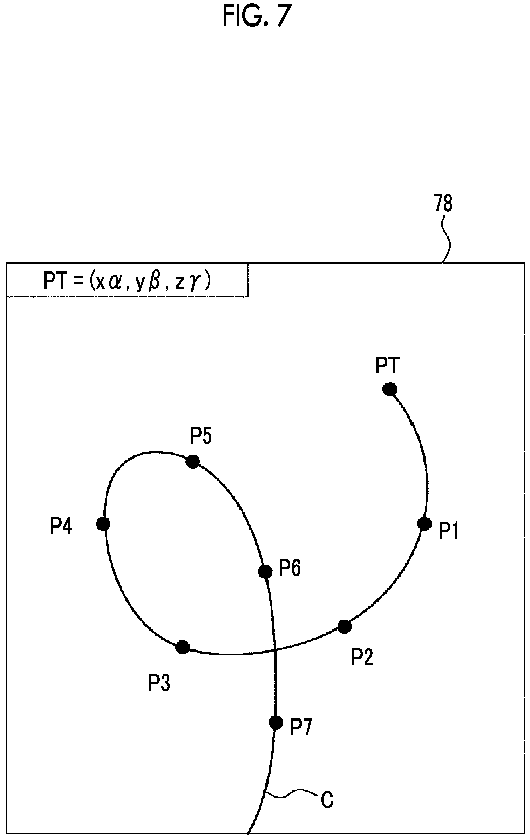

Fig. 7 is an explanatory diagram for describing an example of a shape detection process of the insertion part 17 by the insertion part shape detection unit 73. As shown in Fig. 7, the insertion part shape detection unit 73 performs an interpolation process of performing interpolation on each position with a curve based on the position (here, P1, P2, ...) of each detection coil 25 indicated by the coil position data 76, derives a central axis C of the insertion part 17, generates insertion part shape data 78 showing the shape of the insertion part 17. The interpolation process of performing interpolation with a curve is, for example, Bezier curve interpolation. The insertion part shape data 78 includes a distal end position PT of the distal end part 23 of the insertion part 17.

In Fig. 3, the insertion part shape detection unit 73 outputs the insertion part shape data 78 to the image generation unit 77. The insertion state detection unit 70 repeatedly performs a determination process by the determination unit 72A, a position detection process of detecting the coil position data 76 of each detection coil 25, a shape detection process of the insertion part 17, and an output of the insertion part shape data 78 each time the image signal acquisition unit 68 acquires new frame image signal 61.

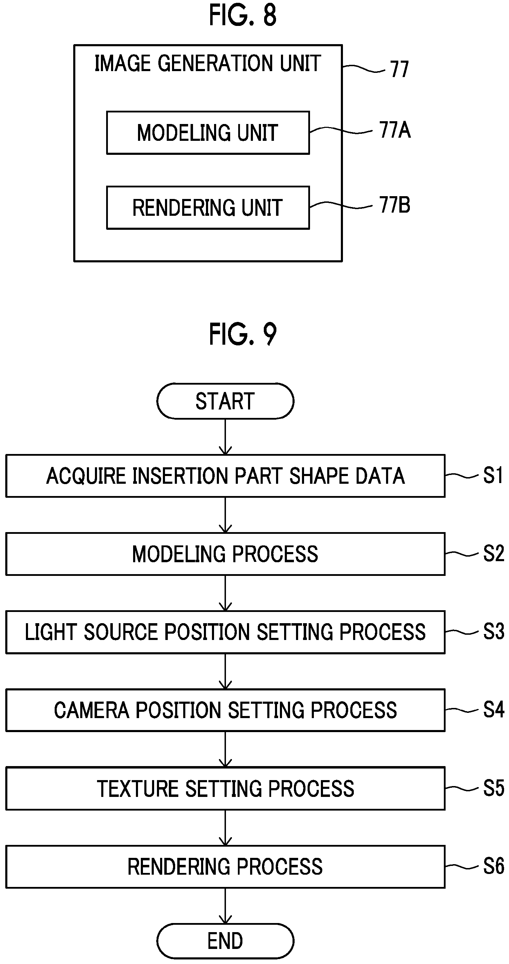

The image generation unit 77 generates the shape display image 42 based on the insertion part shape data 78. As shown in Fig. 8, a modeling unit 77A and a rendering unit 77B are provided in the image generation unit 77. As will be described in detail below, in the image generation unit 77, the modeling unit 77A generates a 3 dimension (D) model of the insertion part 17 based on the insertion part shape data 78, and the rendering unit 77B performs a rendering process on the 3D model of the insertion part 17, so that the 2 dimension (D) shape display image 42 showing the shape of the insertion part 17 is generated. The image generation unit 77 outputs data of the generated shape display image 42 to the processor device 14.

The image generation unit 77 updates the shape display image 42 each time new insertion part shape data 78 is input. Further, the image generation unit 77 outputs the updated data of the shape display image 42 to the processor device 14 each time the shape display image 42 is updated.

The navigation device 12 is an example of an endoscope shape display control device according to the technology of the present disclosure that performs a display control of the shape display image 42. As described above, the magnetic field measurement data 55 is data obtained by using the plurality of detection coils 25 which are an example of a plurality of magnetic field detection elements according to the technology of the present disclosure provided in the insertion part 17 of the endoscope 10. The insertion part shape data 78 is information based on the magnetic field measurement data 55, and is an example of information according to the technology of the present disclosure for imaging the shape of the insertion part 17 of the endoscope 10 inserted in the subject H, which is an example of the subject. The image generation unit 77 generates the shape display image 42 obtained by imaging the shape of the insertion part 17 based on the insertion part shape data 78. That is, the image generation unit 77 is an example of an image generation unit according to the technology of the present disclosure, and is also an example of an information acquisition unit that acquires the information for imaging the shape of the insertion part.

The display output unit 74 outputs the frame image signal 61 previously input from the image signal acquisition unit 68 described above and the data of the shape display image 42 input from the image generation unit 77 to the processor device 14 via communication interfaces 80A and 80B. In this case, the display output unit 74 associates the frame image signal 61 with the data of the shape display image 42 that corresponds in time with the frame image signal 61, and outputs the data to the processor device 14.

### <Processor Device>

The processor device 14 has a display input unit 82 and a display control unit 83. The display input unit 82 sequentially outputs, to the display control unit 83, the data of the frame image signal 61 and the shape display image 42 sequentially input from the display output unit 74 via the communication interfaces 80A and 80B.

The display control unit 83 receives the input of the frame image signal 61 from the display input unit 82, and displays the observation image 41 (for example, the motion picture) based on the frame image signal 61 on the monitor 15 (see Figs. 1 and 2). In addition, the display control unit 83 receives input of the data of the shape display image 42 from the display input unit 82 and displays the shape display image 42 (for example, the motion picture) on the monitor 15 (see Figs. 1 and 2).

### [Flow of Generation Process of Shape Display Image]

Fig. 9 is a flowchart for describing a flow of a generation process of the shape display image 42 by the image generation unit 77.

The image generation unit 77 acquires the insertion part shape data (step S1). In the image generation unit 77, the modeling unit 77A performs a modeling process of generating the 3D model of the insertion part 17 based on the insertion part shape data 78 (step S2).

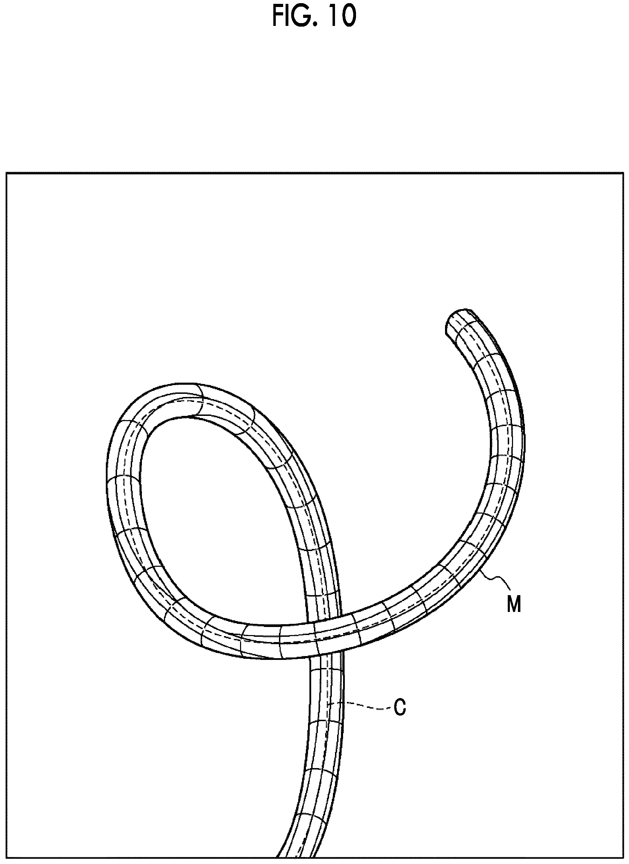

As shown in Fig. 10, in the modeling process, a cylindrical 3D model M having a diameter corresponding to a display scale of the insertion part 17 with the central axis C of the insertion part 17 in the insertion part shape data 78 as a central axis is generated.

Next, the rendering unit 77B executes a light source position setting process of setting the light source position in a case of rendering the 3D model M of the insertion part 17 (step S3). The light source position is set to a position with respect to the upper surface 16A of the bed 16 at which the light is emitted from an upper side in a virtual space in which the 3D model M is generated. Specifically, in a real space, in a case in which the magnetic field generator 13 and the bed 16 are installed on the same floor surface, a horizontal direction of the magnetic field generator 13 and the upper surface 16A of the bed 16 are in a parallel relationship. Therefore, in the virtual space, a plane parallel to the upper surface 16A of the bed 16 is set as an X-Y plane, and a Z-axis orthogonal to the X-Y plane is set. Further, in the virtual space, the light source position is set such that the light is emitted from an upper side of the set Z-axis to a lower side thereof.

Next, the rendering unit 77B executes a camera position setting process of setting a camera position (also referred to as a viewpoint position) in a case of rendering the 3D model M of the insertion part 17 (step S4). The camera position is set to a position with respect to the upper surface 16A of the bed 16 in the virtual space in which the 3D model M of the insertion part 17 is observed from the upper side of the Z-axis in the same manner as the light source position.

Next, the rendering unit 77B executes a texture setting process of finishing a surface of the 3D model M in a case of rendering the 3D model M of the insertion part 17 (step S5). In the texture setting process, a process of adding a pattern, which is a content of the texture, is performed on the surface of the insertion part 17. Here, the pattern refers to a display aspect other than a plain single color. The pattern of the present embodiment is a gradation that is changed continuously from green to blue from the distal end side to the proximal end side of the insertion part 17.

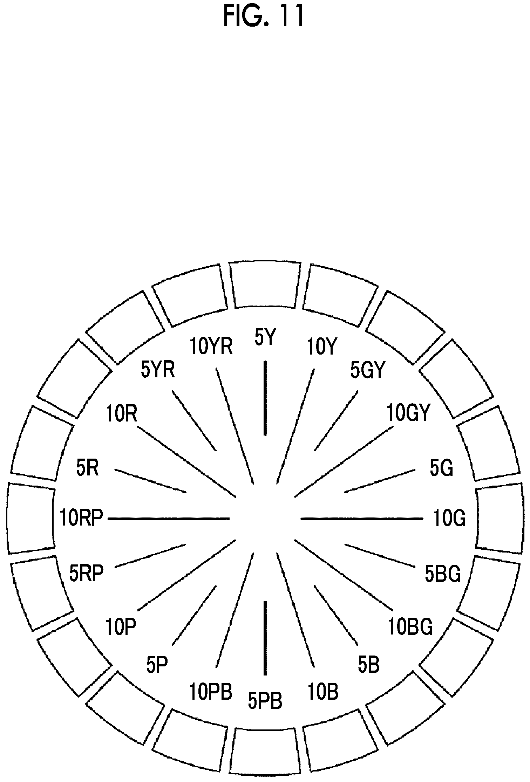

The definition of color will be described using a hue circle of a Munsell display system shown in Fig. 11. The hue circle represents the classification of tint of chromatic color. For example, it is a Munsell hue circle divided into 20 colors. In Fig. 11, symbols means red (R), yellow-red (YR), yellow (Y), green-yellow (GY), green (G), blue-green (BG), blue (B), purple-blue (PB), purple (P), and red purple (RP), respectively. In the hue circle shown in Fig. 11, hues obtained by further dividing each of these 10 types of hues into two (for example, 5R and 10R for R) are displayed.

The green includes green-yellow (GY) and blue-green (BG) that are adjacent to each other on the right and left of green (G) in the hue circle. In the hue circle of Fig. 11, colors from 5 GY to 10 BG correspond to green. Blue includes blue-green (BG) and purple-blue (PB) that are adjacent to each other on the right and left of blue (B) in the hue circle. In the hue circle of Fig. 11, the colors from 5BG to 10PB correspond to blue. As described above, in the hue circle, green refers to a color containing a G element, and blue refers to a color containing a B element. The same applies to the definitions of other colors, such as red and yellow.

Finally, the rendering unit 77B adds a gradation pattern to the surface of the 3D model M based on the set light source position and the set camera position. In addition, the surface of the 3D model M added with the pattern is subjected to a shading process of performing shading corresponding to the light source. As described above, the rendering unit 77B performs the rendering process of generating the 2D shape display image 42 showing the shape of the insertion part 17 (step S6).

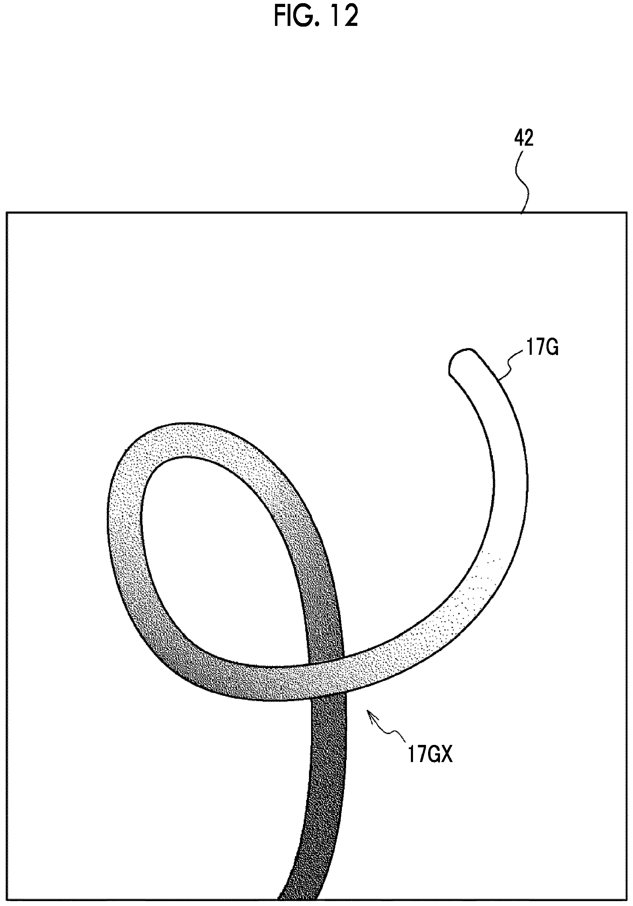

As a result, the shape display image 42 is generated, as shown in Fig. 12. The shape display image 42 is configured to include an image showing the insertion part 17 and an image, which is a background thereof. In the following, the insertion part 17 in the shape display image 42, or more accurately, the image of the insertion part 17 in the shape display image 42, is simply referred to as the insertion part for simplification, and for distinguish from the actual insertion part 17, a reference numeral 17G is added and is referred to as an insertion part 17G In the shape display image 42, the surface of the insertion part 17G is displayed in the display aspect in which the gradation that is changed continuously from green to blue is added from the distal end side to the proximal end side of the insertion part 17G

The generation process of the shape display image 42 is repeated each time the insertion part shape data 78 is updated.

### [Operation and Effect]

In the large intestine examination, since a route of the large intestine is complicated, the insertion part 17 of the endoscope 10 may be bent in a loop shape in the body of the subject H. In this case, different portions of the insertion part 17G intersect with each other in the shape display image 42 depending on the camera position set in the rendering process.

In addition, depending on the display magnification of the shape display image 42, the insertion part 17G may be displayed in a thin line shape in the shape display image 42. Therefore, in a case in which the entire insertion part 17G is displayed in a plain single color (for example, black), there is a problem that it is difficult for a user to instantaneously determine the front and rear of an intersection portion 17GX even in a case in which the intersection portion 17GX shown in Fig. 12 is subjected to a shade line process or a hidden surface process.

On the other hand, in the endoscope system 9 according to the present disclosure, as described above, in the shape display image 42, the insertion part 17G is displayed by being added with the gradation pattern which is changed continuously from green to blue from the distal end side to the proximal end side of the insertion part 17G As a result, the color of the insertion part 17G is different between the front and rear of the intersection portion 17GX, so that it is possible to easily determine the front and rear of the intersection portion 17GX. As described above, according to the technology of the present disclosure, it is easy to visually recognize an overlapping condition of the insertion parts 17G in the shape display image 42.

In the present embodiment, the gradation pattern is added to the entire region of the insertion part 17G displayed on the shape display image 42. Therefore, no matter where in the entire region of the displayed insertion part 17G is the intersection portion 17GX, it is possible to easily determine the front and rear of the intersection portion 17GX.

Note that in the present embodiment, the entire region of the displayed insertion part 17G is added with the gradation pattern, but the entire region does not necessarily have to be added with the gradation pattern. In a case in which the intersection portion 17GX is hardly generated on the distal end side and the proximal end side, it is not necessary to add the gradation pattern to a partial region of the distal end side and the proximal end side. For example, in the entire region of the insertion part 17G displayed on the shape display image 42, a region having a length of 20% from the distal end and a region having a length of 20% from the proximal end may be used as regions without the gradation pattern.

In addition, by making the pattern added to the insertion part 17G the gradation pattern in which the color is changed continuously from the distal end side to the proximal end side of the insertion part 17G, the image can be more natural than a case in which the color is changed stepwise.

In addition, by displaying the insertion part 17G in the display aspect of the gradation that is changed continuously from green to blue, which is a combination of cold colors, the effect of suppressing irritation to the eyes can be expected as compared with a case in which the insertion part 17G is displayed by colors of a warm color system, such as red, orange, and yellow.

### [Modification Example]

The embodiment described above is an example, and various modification examples are possible as shown below.

In the embodiment described above, the gradation that is changed from green to blue has been described as an example of the gradation, but a combination of a plurality of changing colors may be other than the colors of the example described above. For example, it may be a gradation that is changed from red to orange. In addition, the color of the gradation may be changed in accordance with the user's setting.

In the embodiment described above, the gradation pattern in which the color is changed continuously has been described as the pattern to be added to the surface of the insertion part 17G in the shape display image 42, but the pattern may be other than the gradation.

In a case in which the surface of the insertion part 17G is displayed by being added with the pattern, it is possible to easily determine the front and rear of the intersection portion 17GX as compared with a case in which the surface of the insertion part 17G is displayed in a plain single color.

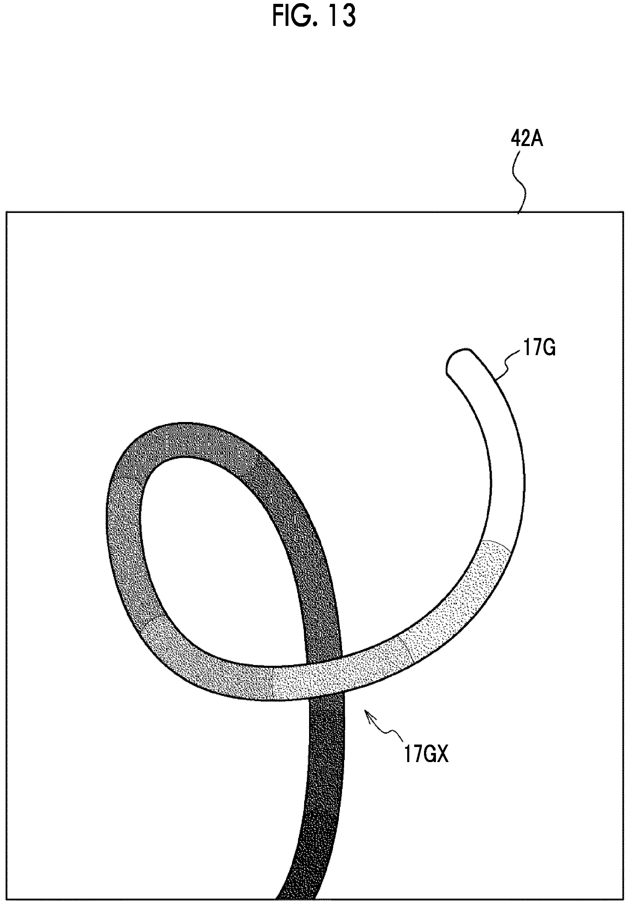

For example, as shown in Fig. 13, the gradation pattern does not have to be an aspect in which the color is changed continuously, but an aspect in which the color is changed stepwise may be adopted.

In a shape display image 42A shown in Fig. 13, the surface of the insertion part 17G is added with the gradation pattern that is changed stepwise to a different color from the distal end side to the proximal end side. Even in a case in which the color is not changed continuously, the colors of the insertion part 17G have different color scheme in the front and rear the intersection portion 17GX, so that it is possible to easily determine the front and rear of the intersection portion 17GX. It is needless to say that the gradation in which the color is changed continuously is preferable in that the color change is natural and the appearance is good.

In addition, the pattern may be a pattern other than the gradation, and may be a pattern in which a plurality of colors are combined. Here, a plurality of colors means, for example, a combination of colors in which at least one of lightness, chroma saturation, or hue is different from each other.

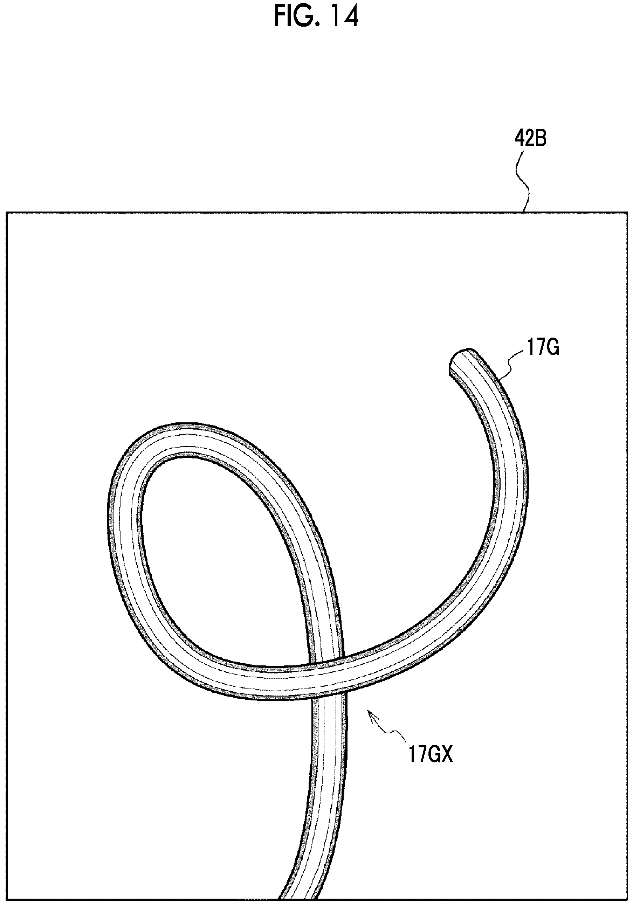

Fig. 14 shows a shape display image 42B according to a non-claimed example, wherein the pattern may be a vertical stripe pattern of a plurality of colors which extends in the longitudinal direction of the insertion part 17G. Even in this example, since the vertical stripes intersect with each other in the front and rear of the intersection portion 17GX, it is possible to easily determine the front and rear of the intersection portion 17GX.

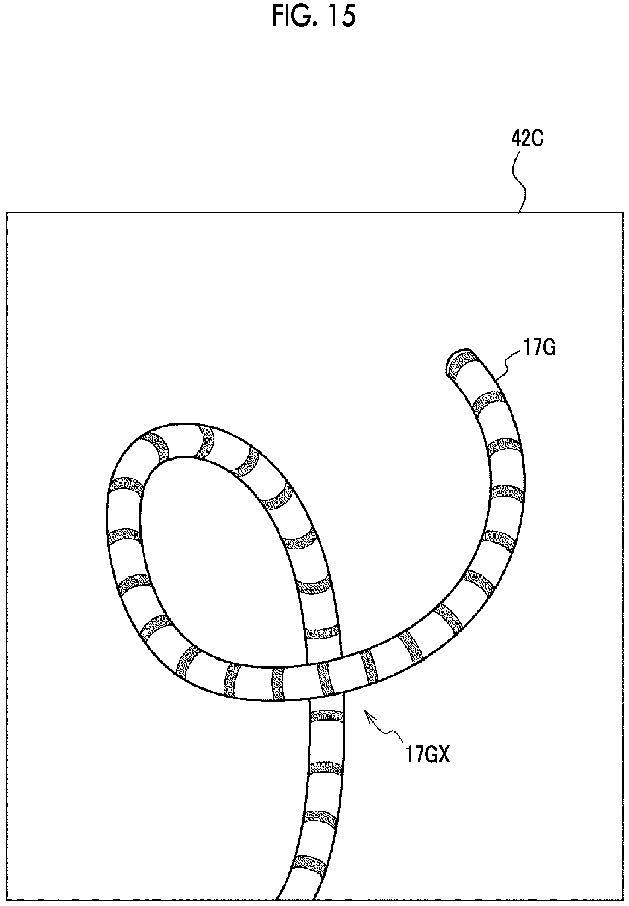

Fig. 15 shows a shape display image 42C according to a non-claimed example, wherein the pattern may be a horizontal stripe pattern added in a ring shape around an axis of the insertion part 17G. Even in this example, since the horizontal stripes intersect with each other in the front and rear of the intersection portion 17GX, it is possible to easily determine the front and rear of the intersection portion 17GX.

Such a vertical stripe pattern and such a horizontal stripe pattern may be constituted by pinstripes, or the vertical stripe pattern and the horizontal stripe pattern may be constituted by a plurality of strip-shaped stripes having a width and the plurality of stripes are color-coded with different colors.

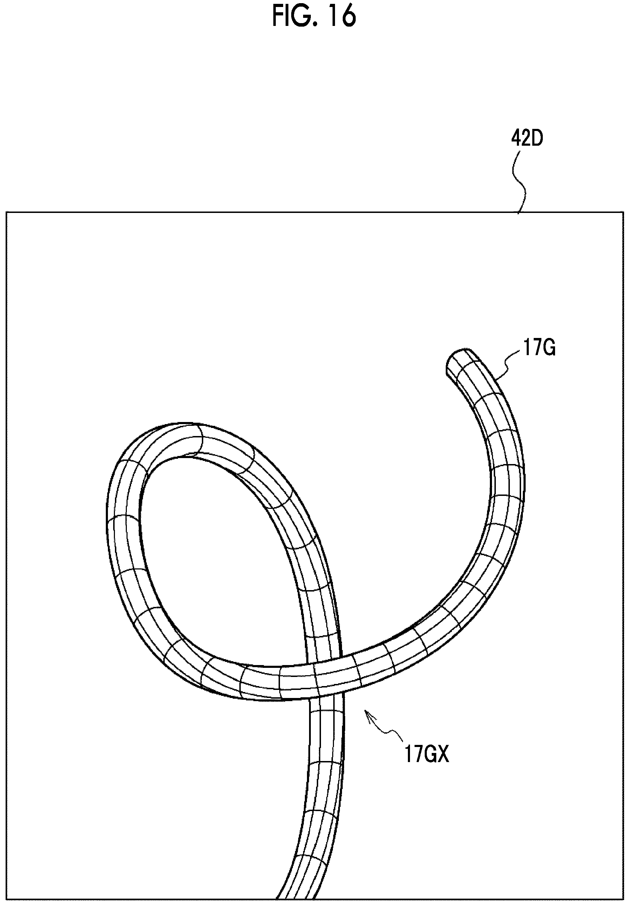

According to a non-claimed example, a wire frame pattern may be used as in a shape display image 42D shown in Fig. 16. Note that the wire frame expresses a three-dimension object by connecting a plurality of points having three-dimension coordinates in accordance with a certain rule. The wire frame pattern is a display aspect in which the wire frame is displayed as the pattern on the surface of the insertion part 17G. Even with such a pattern, the same effect as in the example described above can be obtained.

In the embodiment described above, regarding the light source position and the camera position in a case of performing the rendering process, a case will be described in which, in a case in which the plane parallel to the upper surface 16A of the bed 16 is defined as the X-Y plane, a light irradiation direction and a viewpoint direction are set to a state of being from the upper side of the Z-axis to the lower side thereof. However, the light source position and the camera position are not limited to the above, and may be set in any direction based on an instruction input of the user. Examples of the direction in which the viewpoint direction is from the upper side of the Z-axis of the bed 16 to the lower side thereof include the following two directions. One direction is the viewpoint of observing the upper surface 16A of the bed 16 from directly above the bed 16, that is, from a normal direction to the X-Y plane. The other direction is a viewpoint of a bird's-eye view of the upper surface 16A of the bed 16 from an oblique direction of the bed 16. In addition to these, there may be an observation viewpoint on a side of the bed 16, that is, parallel to the X-Y plane. In addition, a plurality of viewpoints may be switched and parallel viewpoints may be set.

In addition, in the embodiment described above, the example has been described in which each generation coil 39 generates the magnetic field MF having the same frequency, but the frequencies of the magnetic fields MF generated by the generation coils 39 may be different.

In addition, in the embodiment described above, the example has been described in which the magnetic field measurement unit including the magnetic field measurement control unit 58 is disposed in the endoscope 10 and the magnetic field generation control unit 71 is disposed in the navigation device 12, on the contrary, the magnetic field generation control unit 71 may be disposed in the endoscope 10, and the magnetic field measurement unit may be disposed in the navigation device 12.

In the embodiment described above, for example, as a hardware structure of the processing unit that executes various processes, such as the image generation unit 77 which is an example of the information acquisition unit and the image generation unit, various processors in the following can be used. The various processors include the central processing unit (CPU) that is a general-purpose processor executing the software and functioning as the various processing units, as well as a programmable logic device (PLD) that is a processor of which a circuit configuration can be changed after manufacture, such as a field programmable gate array (FPGA) and/or a dedicated electric circuit that is a processor having a circuit configuration that is designed for exclusive use in order to execute a specific process, such as an application specific integrated circuit (ASIC).

One processing unit may be configured by one of the various processors, or may be a combination of two or more processors of the same type or different types (for example, a combination of a plurality of FPGAs and/or a combination of a CPU and an FPGA). In this way, as the hardware structure, various processing units are configured by one or more of various processors described above.

Moreover, as the hardware structure of these various processors, more specifically, it is possible to use an electrical circuit (circuitry) in which circuit elements such as semiconductor elements are combined.

An endoscope display control device having the information acquisition unit and the image generation unit may be realized by the computer of which the processor is the CPU.

A program for causing the CPU to function as the information acquisition unit and the image generation unit is an operation program of the endoscope display control device. The technology of the present disclosure extends to a computer-readable storage medium that stores the operation program non-transitorily, in addition to the operation program of the endoscope display control device.

In the present specification, "A and/or B" is synonymous with "at least one of A or B". That is, "A and/or B" means that it may be only A, only B, or a combination of A and B. In addition, in the present specification, in a case in which three or more matters are associated and expressed by "and/or", the same concept as "A and/or B" is applied.

The contents described and shown above are the detailed description of the parts relating to the technology of the present disclosure, and are merely an example of the technology of the present disclosure. For example, the above description of the configuration, the function, the action, and the effect are the description of examples of the configuration, the function, the action, and the effect of the parts relating to the technology of the present disclosure. Therefore, it is needless to say that unnecessary parts may be deleted, new elements may be added, or replacements may be made with respect to the contents described and shown above within a range that does not deviate from the gist of the technology of the present disclosure. In addition, in order to avoid complications and facilitate understanding of the parts relating to the technology of the present disclosure, in the contents described and shown above, the description of common general knowledge and the like that do not particularly require description for enabling the implementation of the technology of the present disclosure are omitted.

## Claims

1. An endoscope shape display control device comprising:
at least one processor (14),
wherein the at least one processor (14) is configured
to acquire information detected by a navigation device (12) for imaging a shape of an insertion part (17) of an endoscope (10) inserted in a subject (H), and
to display on a monitor (15), in a case of generating a shape display image (42) in which the shape of the insertion part (17) is displayed based on the acquired information, the insertion part (17) with a pattern added on a surface thereof in the shape display image,
wherein the pattern is a gradation in which a color is changed continuously or stepwise from one of a distal end side or a proximal end side of the insertion part (17) to the other thereof, and
wherein the gradation is changed from green to blue.

2. The endoscope shape display control device according to claim 1, wherein the at least one processor is configured to add the pattern to an entire region of the insertion part (17) displayed in the shape display image (42).

3. The endoscope shape display control device according to any one of the preceding claims, wherein the at least one processor (14) is configured to perform a shading process on the insertion part (17) in addition to the pattern.

4. The endoscope shape display control device according to any one of the preceding claims, wherein the information for imaging the shape of the insertion part (17) is information based on magnetic field measurement data obtained by using a plurality of magnetic field generation elements (39) or a plurality of magnetic field detection elements (25) provided in the insertion part (17).

5. An operation method of an endoscope shape display control device, the method comprising:
an information acquisition step performed by at least one processor of acquiring information detected by a navigation device (12) for imaging a shape of an insertion part (17) of an endoscope (10) inserted in a subject (H); and
an image generation step performed by the at least one processor of generating a shape display image (42) displayed on a monitor (15) in which the shape of the insertion part (17) is displayed based on the acquired information and displaying the insertion part (17) with a pattern added on a surface thereof in the shape display image (42),
wherein the pattern is a gradation in which a color is changed continuously or stepwise from one of a distal end side or a proximal end side of the insertion part (17) to the other thereof, and
wherein the gradation is changed from green to blue.

6. An operation program of an endoscope shape display control device, the program causing a computer to function as:
an information acquisition unit that acquires information detected by a navigation device (12) for imaging a shape of an insertion part (17) of an endoscope (10) inserted in a subject (H); and
an image generation unit that generates a shape display image (42) displayed on a monitor (15) in which the shape of the insertion part (17) is displayed based on the acquired information, and displays the insertion part (17) with a pattern added on a surface thereof in the shape display image (42),
wherein the pattern is a gradation in which a color is changed continuously or stepwise from one of a distal end side or a proximal end side of the insertion part (17) to the other thereof, and
wherein the gradation is changed from green to blue.

## Patentansprüche

1. Steuervorrichtung für die Formanzeige eines Endoskops bzw. Endoskopformanzeige-Steuervorrichtung, umfassend:
mindestens einen Prozessor (14),
wobei der mindestens eine Prozessor (14) konfiguriert ist
zum Erfassen von Informationen, die von einer Navigationsvorrichtung (12) erfasst werden, um eine Form eines Einführungsteils (17) eines in ein Subjekt bzw. eine Person (H) eingeführten Endoskops (10) abzubilden, und
zum Anzeigen auf einem Monitor (15) in einem Fall des Erzeugens eines Formanzeigebildes (42), in dem die Form des Einführungsteils (17) basierend auf den erfassten Informationen angezeigt wird, des Einführungsteils (17) mit einem Muster, das auf einer Oberfläche davon in dem Formanzeigebild hinzugefügt ist,
wobei das Muster eine Abstufung ist, bei der eine Farbe kontinuierlich oder schrittweise von einer distalen Endseite oder einer proximalen Endseite des Einführungsteils (17) zu der anderen davon geändert wird, und
wobei die Abstufung von Grün zu Blau geändert wird.

2. Die Endoskopformanzeige-Steuervorrichtung nach Anspruch 1, wobei der mindestens eine Prozessor so konfiguriert ist, dass er das Muster zu einem gesamten Bereich des Einführteils (17) hinzufügt, der in dem Formanzeigebild (42) angezeigt wird.

3. Endoskopformanzeige-Steuerungsvorrichtung nach einem der vorhergehenden Ansprüche, wobei der mindestens eine Prozessor (14) so konfiguriert ist, dass er zusätzlich zu dem Muster einen Schattierungsprozess auf dem Einführungssteil (17) durchführt.

4. Endoskopformanzeige-Steuerungsvorrichtung nach einem der vorhergehenden Ansprüche, wobei die Informationen zur Abbildung der Form des Einführungsteils (17) Informationen sind, die auf Magnetfeldmessdaten basieren, die unter Verwendung einer Vielzahl von Magnetfelderzeugungselementen (39) oder einer Vielzahl von Magnetfelderfassungselementen (25), die in dem Einführungsteil (17) vorgesehen sind, erhalten werden.

5. Betriebsverfahren für eine Endoskopformanzeige-Steuerungsvorrichtung, wobei das Verfahren umfasst:
einen Informationserfassungsschritt, der von mindestens einem Prozessor durchgeführt wird, um Informationen zu erfassen, die von einer Navigationsvorrichtung (12) erfasst werden, um eine Form eines Einführungsteils (17) eines in ein Subjekt (H) eingeführten Endoskops (10) abzubilden; und
einen Bilderzeugungsschritt, der von dem mindestens einen Prozessor ausgeführt wird, um ein Formanzeigebild (42) zu erzeugen, das auf einem Monitor (15) angezeigt wird, in dem die Form des Einführungsteils (17) auf der Grundlage der erfassten Informationen angezeigt wird, und um das Einführungsteil (17) mit einem Muster anzuzeigen, das auf einer Oberfläche desselben in dem Formanzeigebild (42) hinzugefügt ist,
wobei das Muster eine Abstufung ist, bei der eine Farbe kontinuierlich oder schrittweise von einer distalen Endseite oder einer proximalen Endseite des Einführungsteils (17) zu der anderen davon geändert wird, und
wobei die Abstufung von Grün zu Blau geändert wird.

6. Ein Betriebsprogramm einer Endoskopformanzeige-Steuerungsvorrichtung, wobei das Programm einen Computer veranlasst, zu funktionieren bzw. zu wirken als:
eine Informationserfassungseinheit, die Informationen erfasst, die von einer Navigationsvorrichtung (12) erfasst werden, um eine Form eines Einführungsteils (17) eines in ein Subjekt (H) eingeführten Endoskops (10) abzubilden; und
eine Bilderzeugungseinheit, die ein Formanzeigebild (42) erzeugt, das auf einem Monitor (15) angezeigt wird, in dem die Form des Einführungsteils (17) auf der Grundlage der erfassten Informationen angezeigt wird, und das Einführungsteil (17) mit einem Muster anzeigt, das auf einer Oberfläche desselben in dem Formanzeigebild (42) hinzugefügt ist,
wobei das Muster eine Abstufung ist, bei der eine Farbe kontinuierlich oder schrittweise von einer distalen Endseite oder einer proximalen Endseite des Einführungsteils (17) zu der anderen davon geändert wird, und
wobei die Abstufung von Grün zu Blau geändert wird.

## Revendications

1. Dispositif de contrôle de l'affichage de la forme de l'endoscope comprenant
au moins un processeur (14),
dans lequel l'au moins un processeur (14) est configuré pour
acquérir des informations détectées par un dispositif de navigation (12) pour imager la forme d'une partie d'insertion (17) d'un endoscope (10) inséré dans un sujet (H), et
afficher sur un moniteur (15), en cas de génération d'une image d'affichage de forme (42) dans laquelle la forme de la pièce d'insertion (17) est affichée sur la base des informations acquises, la pièce d'insertion (17) avec un motif ajouté sur une de ses surfaces dans l'image d'affichage de forme,
dans lequel le motif est un dégradé dans lequel une couleur est modifiée de manière continue ou par étapes d'un côté de l'extrémité distale ou d'un côté de l'extrémité proximale de la partie d'insertion (17) à l'autre, et
dans lequel la gradation passe du vert au bleu.

2. Le dispositif de contrôle de l'affichage de la forme de l'endoscope selon la revendication 1, dans lequel l'au moins un processeur est configuré pour ajouter le motif à une région entière de la partie d'insertion (17) affichée dans l'image d'affichage de la forme (42).

3. Le dispositif de contrôle de l'affichage de la forme de l'endoscope selon l'une quelconque des revendications précédentes, dans lequel l'au moins un processeur (14) est configuré pour effectuer un processus d'ombrage sur la partie d'insertion (17) en plus du motif.

4. Le dispositif de contrôle de l'affichage de la forme de l'endoscope selon l'une quelconque des revendications précédentes, dans lequel les informations pour l'imagerie de la forme de la partie d'insertion (17) sont des informations basées sur des données de mesure du champ magnétique obtenues en utilisant une pluralité d'éléments de génération de champ magnétique (39) ou une pluralité d'éléments de détection de champ magnétique (25) fournis dans la partie d'insertion (17).

5. Méthode de fonctionnement d'un dispositif de contrôle de l'affichage de la forme d'un endoscope, comprenant :
une étape d'acquisition d'informations réalisée par au moins un processeur consistant à acquérir des informations détectées par un dispositif de navigation (12) pour imager la forme d'une partie d'insertion (17) d'un endoscope (10) inséré dans un sujet (H) ; et
une étape de génération d'images réalisée par l'au moins un processeur consistant à générer une image d'affichage de forme (42) affichée sur un moniteur (15) dans laquelle la forme de la pièce d'insertion (17) est affichée sur la base des informations acquises et à afficher la pièce d'insertion (17) avec un motif ajouté sur une de ses surfaces dans l'image d'affichage de forme (42),
dans lequel le motif est un dégradé dans lequel une couleur est modifiée de manière continue ou par étapes d'un côté de l'extrémité distale ou d'un côté de l'extrémité proximale de la partie d'insertion (17) à l'autre, et
dans lequel la gradation passe du vert au bleu.

6. Programme de fonctionnement d'un dispositif de contrôle de l'affichage de la forme d'un endoscope, le programme permettant à un ordinateur de fonctionner comme suit :
une unité d'acquisition d'informations qui acquiert des informations détectées par un dispositif de navigation (12) pour imager la forme d'une partie d'insertion (17) d'un endoscope (10) inséré dans un sujet (H) ; et
une unité de génération d'images qui génère une image d'affichage de forme (42) affichée sur un moniteur (15) dans laquelle la forme de la pièce d'insertion (17) est affichée sur la base des informations acquises, et affiche la pièce d'insertion (17) avec un motif ajouté sur une de ses surfaces dans l'image d'affichage de forme (42),
dans lequel le motif est un dégradé dans lequel une couleur est modifiée de manière continue ou par étapes d'un côté de l'extrémité distale ou d'un côté de l'extrémité proximale de la partie d'insertion (17) à l'autre, et
dans lequel la gradation passe du vert au bleu.
